# EUROPEAN PATENT SPECIFICATION

(11) **EP 1 183 035 B1**
(45) Date of publication and mention of the grant of the patent: **03.12.2014**
(21) Application number: 00935910.0
(22) Date of filing: 11.05.2000
(51) Int. Cl.: A61K 35/28, A61P 25/16, A61P 25/28

(54) **BONE MARROW TRANSPLANTATION FOR TREATMENT OF STROKE**
KNOCHENMARKSTRANSPLANTATION ZUR BEHANDLUNG VON SCHLAGANFALL
GREFFE DE MOELLE OSSEUSE POUR TRAITER L'ACCIDENT VASCULAIRE CEREBRAL

(30) Priority: 14.05.1999 US 134344 P
(43) Date of publication of application: 06.03.2002
(73) Proprietor: HENRY FORD HEALTH SYSTEM, Detroit, MI 48202-3450 (US)
(72) Inventor: LI, Yi, Canton, MI 48187 (US); CHOPP, Michael, Southfield, MI 48034 (US)
(74) Representative: Bradbury, Simon Timothy Nicholas
(86) International application number: PCT/US2000/012875
(87) International publication number: WO 2000/069448

(56) References cited:
- WO-A-00/50568
- WO-A-99/43286
- WO-A-99/56759
- US-A- 5 690 927
- US-A- 5 750 376
- US-A- 5 817 773
- US-A- 5 851 832
- AZIZI S AUSIM ET AL: "Engraftment and migration of human bone marrow stromal cells implanted in the brains of albino rats-similarities to astrocyte grafts" PROCEEDINGS OF THE NATIONAL ACADEMY OF SCIENCES OF USA, NATIONAL ACADEMY OF SCIENCE. WASHINGTON, US, vol. 95, no. 7, 31 March 1998 (1998-03-31), pages 3908-3913, XP002199138 ISSN: 0027-8424
- HO S-H ET AL: "INDUCTION OF NG108-15 CELLS DIFFERRENTIATION BY HUMAN BONE MARROW STROMAL CELLS" NEUROREPORT, RAPID COMMUNICATIONS OF OXFORD, OXFORD, GB, vol. 9, no. 7, 11 May 1998 (1998-05-11), pages 1365-1369, XP001042196 ISSN: 0959-4965
- LOZANO A M ET AL: "A CONVENIENT IN VITRO ASSAY FOR THE INHIBITION OF NEURITE OUTGROWTH BY ADULT MAMMALIAN CNS MYELIN USING IMMORTALIZED NEURONALCELLS" JOURNAL OF NEUROSCIENCE METHODS, ELSEVIER SCIENCE PUBLISHER B.V., AMSTERDAM, NL, vol. 63, no. 1/2, 1 December 1995 (1995-12-01), pages 23-28, XP000600713 ISSN: 0165-0270
- WALKLEY S U ET AL: "Bone marrow transplantation corrects the enzyme defect in neurons of the central nervous system in a lysosomal storage disease" PROCEEDINGS OF THE NATIONAL ACADEMY OF SCIENCES OF USA, NATIONAL ACADEMY OF SCIENCE. WASHINGTON, US, vol. 91, no. 8, 1994, pages 2970-2974, XP002182077 ISSN: 0027-8424
- FLAX JONATHAN D ET AL: "Engraftable human neural stem cells respond to developmental cues, replace neurons, and express foreign genes" NATURE BIOTECHNOLOGY, NATURE PUBLISHING, US, vol. 16, no. 11, November 1998 (1998-11), pages 1033-1039, XP002197601 ISSN: 1087-0156
- JACKOWSKI ET AL.: 'Neural injury repair: hope for the future as barriers to effective CNS regeneration become clearer' BRITISH J. NEUROSURGERY, vol. 9, 1995, pages 303 - 317, XP002931089

## Description

### CROSS REFERENCE TO RELATED APPLICATIONS

This application is a conversion of United States Provisional Patent Application No. 60/134,344, filed May 14, 1999, incorporated herein by reference.

### TECHNICAL FIELD

The present invention relates to a treatment of neural injury and neurodegenerative diseases. More specifically, the present invention relates to the use of bone stromal marrow cells for the treatment of stroke.

### BACKGROUND ART

Intracerebral transplantation of donor cells from embryonic tissue may promote neurogenesis (Synder et al. 1997). Intrastriatal fetal graft has been used to reconstruct damaged basal ganglia circuits and to ameliorate behavioural deficits in an animal model of ischemia (Goto et al. 1997). Fetal hematopoietic stem cells (HSCs) transplanted into the adult organism or adult HSCs transplanted into an embryo results in a chimera that reflects the endogenous cells within the microenvironment into which the cells were seeded (Geiger et al. 1998). Pluripotent stem cells are harboured in the adult CNS and the adult brain can form new neurons (Gage 1998; Kempermann and Gage, 1998).

The concept of transplantation of bone marrow has been studied by others. For example, in the Azzizi et al. reference the investigators transplant human bone marrow stromal cells in the brains of albino rats. Their primary observations were that human mesenchymal cells can engraft, migrate and survive in a manner similar to rat astrocytes. Further in the manuscript by Eglitis and Mwzey there is shown that the bone marrow cells when implanted into the brain of adult mice can differentiate into microglia and macroglia. Again, this occurred when transplanted into the brain of normal mice. There two papers were used to support a hypothesis that some astroglia arise from a precursor cell that is a normal constituent of bone marrow. However, there has been no study showing that bone marrow cells differentiate into neurons. Further, there has been no study that this would occur in a damaged brain or spinal cord and in neurodegenerative disease. In addition, there have been no data that treatment of neural injury (stroke, traumatic, brain injury, spinal cord injury) and neurodegenerative disease (Parkinson's) with bone marrow cells improves functional outcome.

### SUMMARY OF THE INVENTION

According to the present invention, there is provided use of bone marrow stromal cells in the manufacture of a medicament for the treatment of stroke by transplanting the cells intravascularly by intracarotid administration or intravenous administration. Suitably the use is to activate endogenous central nervous system stem cells to differentiate into neurons in an injured brain.

Whole bone marrow and cellular components of bone marrow have been employed (i.e. mesenchymal stem cells, MSCs; hematopoietic stem cells HSGs) to treat stroke (rat, mouse) and traumatic brain injury (rat). Cellular components of bone marrow were cultured in a special medium and in medium containing neurotrophins (NGF, BDNF). Cells were injected either directly into brain, into the internal carotid artery or into a femoral vein. Outcome measures were: double staining immunohistochemistry to morphologically identify phenotypic transformation of bone marrow cells and behavioural and functional tests to identify neurological deficits. Our data demonstrate that treatment of stroke, spinal cord injury, or traumatic brain injury with whole bone marrow or cellular components significantly reduces functional deficits. Bone marrow cells also express phenotypes of parenchymal cells. In addition, mice treated with the neurotoxin MPTP to induce symptoms of Parkinson' disease, were treated with bone marrow cells delivered intracerebrally. Parkinson's symptoms were significantly reduced in mice treated with bone marrow cells. These data demonstrate that bone marrow cells can be employed to treat neural injury and neurodegenerative disease.

Major and novel contributions to this field are: the culturing of bone marrow cells in neurotrophins, the intraparenchymal and intravascular administration of these cells (cultured with growth factor or not) for therapy and the treatment of stroke, trauma and Parkinson's disease with bone marrow.

Also developed is an aggregate, composed of neural stem cells from the fetal neurosphere, mesenchymal stem cells from adult bone marrow and cerebro-spinal fluid from adult Wistar rats (called NMCspheres). These NMCspheres have been successfully used to treat stroke and brain trauma, and can be employed to treat neurodegenerative disease.

### BRIEF DESCRIPTION OF THE DRAWINGS

Other advantages of the present invention will be readily appreciated as the same becomes better understood by reference to the following detailed description when considered in connection with the accompanying drawings wherein:
FIGURES. 1A-B are diagrams of the three regions of the rat brain after two hours of MCAo with bone marrow transplantation;
FIGURES 2A-L are photographs showing the bone marrow cells in the H&E prepared section in the immunoreactivity of representative proteins in the IBZ of a series of adjacent sections from rats killed four days after bone marrow transplantation (A-H); FIGURE 2I shows the neuronal specific nuclear protein, NeuN; FIGURE 2J shows that the bone marrow transplantation of the cells adjacent to the ependymal cells showing reactivity for the neuronal marker MAP-2; and K-L show that the cells of the SVZ express Neuro D and GFAP protein markers);
FIGURES 3A-H are photographs showing H&E prepared sections of cerebral tissue after MCA transplanted with bone marrow cell transplantation;
FIGURES 3I-J are photographs showing the TUNEL staining showing apoptotic-like cells within the bone marrow grafting at four days;
FIGURES 4A-C show data from the adhesive-removal test, the rotorod-motor test and the neurological severity score, respectively;
FIGURES 5A-B depicts grafts showing that mice treted with transplanted MSC exhibit a significant improvement in the duration on the rotarod and how an improved neurological function compared to vehicle treated animals;

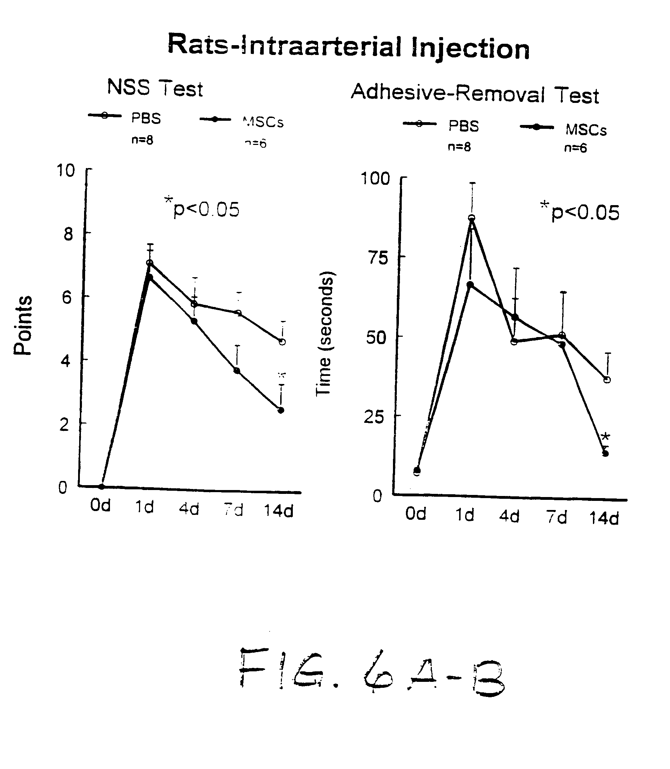

FIGURES 6A-B depict that rats with MSC intraarterial transplantation exhibited significant improvement on the adhesive-removal test and the modified neurological severity scores at 14 days compared with controls;
FIGURES 7A-B depict functional data from rats admiistered MSC intravenously compared to control-ischemia rats;
FIGURE 8 depicts rotarod data from mice subjected to MPTP neurotoxicity;

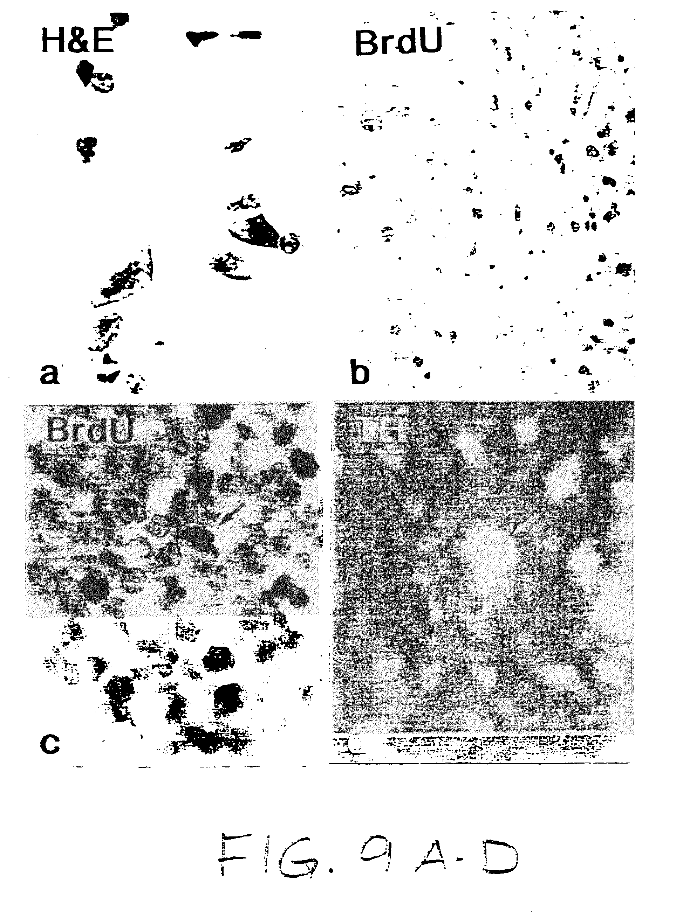

FIGURES 9A-D depicts the morphological changes, i.e. most shrunk pigmented neurons disappeared and only few of them were observed in the substantia nigra at 45 days after MSC transplantation in MPTP-DP mice; viable BrdU immunoreactive cells identified in the injected area and migrated to variable distances into th ehost striatum at 45 days; double staining shows that scattered BrdU reactive cells express TH immunoreactivity within the grafts;

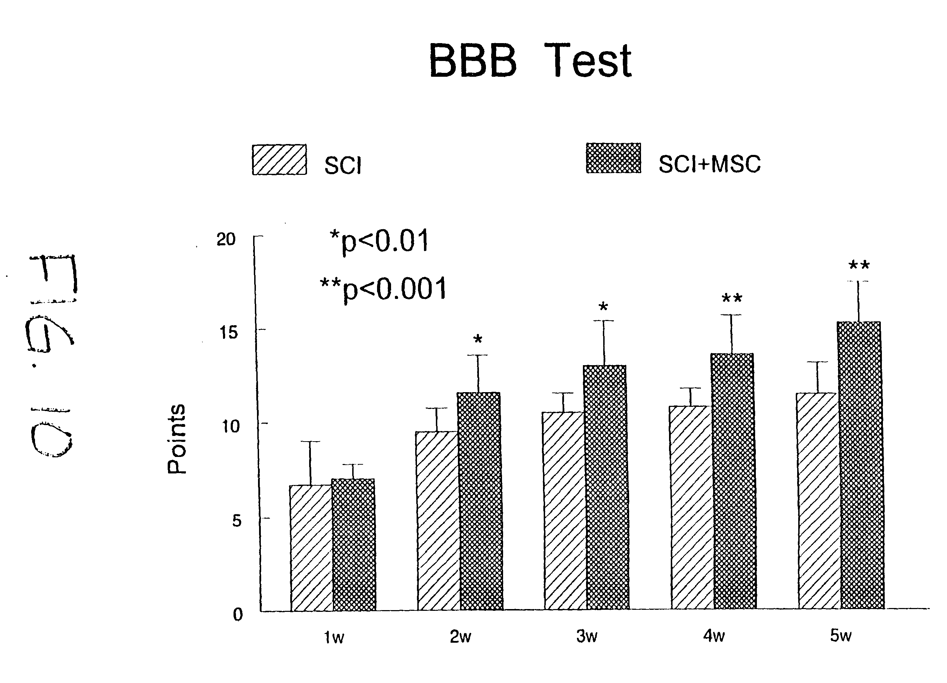

FIGURE 10 shows data from the BBB test from animals subjected to spinal cord injury; and

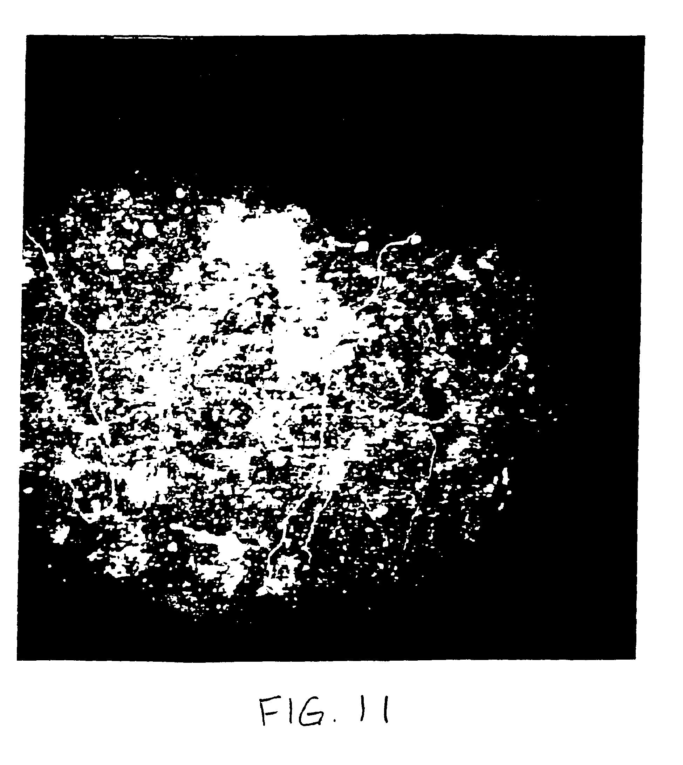

FIGURE 11 is a photograph depicting the composite MSC-neurosphere nine days after cell-neurosphere integration.

### DETAILED DESCRIPTION

Generally, the present invention is useful in a method of treating neural injury and neurodegeneration using bone marrow transplantation. It has been determined that the bone marrow cells differentiate into neurons and other parenchymal cells. Bone marrow cells within injured brain and spinal cord produce an array of cytokines and growth factors. The bone marrow cells activate the endogenous stem cells in the brain, the ependymal cells, to proliferate and to differentiate into parenchymal cells including neurons. New neurons are then present at the dentate gyrus and olfactory bulb and adjacent to the sites of injury. Thus, the bone marrow activates endogenous central nervous system stem cells to differentiate into neurons. The bone marrow cells also produce factors (cytokines and growth factors) that promote repair and plasticity of brain.

Described herein is also a method that employs specific culturing of bone marrow cells, and specific sites of injection of bone marrow. The cells are transplanted into the penumbral tissue, adjacent to a lesion, and not within the lesion. The adjacent tissue to the lesion provides a receptive environment similar to that of a developmental brain, for the survival and differentiation of the bone marrow cells. It is based on this activity that the bone marrow is able to be useful in neural injury and neurodegeneration wherein specific brain or spinal cord damage has occurred. In addition, bone marrow cells are effective in treating neural injury and degeneration when these cells are administered intravascularly, i.e. intraarterially or intravenously. Therefore, after such brain injury, when the brain tissue dies, in an effort to compensate for the lost tissue, the implantation of bone marrow and its derivatives provide sufficient source of cells and activation to promote compensatory responses of the brain to such damage.

The bone marrow is transplanted into the ischemic brain of the rat and mouse, injured rat brain, injured spinal cord and into brain of a Parkinson's mouse. Transplantation into the brain has also been performed with co-transplantation of growth factors (BDNF, NAF). The bone marrow, particularly the MSCs, have been cultured with nerve growth factor (NGF).

Transplantation was performed at various time points (from four hours to two days after stroke, from one to seven days after trauma, seven days after spinal cord injury and fourteen days after initiation of Parkinson's disease in the mouse) after experimental stroke in both the rat and the mouse. The data indicate that the transplantation of bone marrow or components into ischemic brain results in differentiation of the bone marrow cells into the brain parenchymal cells, including neurons. In addition, endogenous brain stem cells are activated to proliferate and to differentiate into parenchymal cells. These cells migrate to different regions within brain including the hippocampus, olfactory bulb and cortex. There is also improved functional outcome in rats treated with bone marrow transplantation cultured with or in combination with growth factors. This model is highly predictive of positive results in higher mammals, including humans. A clinical trial for the treatment of the stroke patient with MSC will be submitted to the Institutional Review Board of Henry Ford Hospital for review.

The above discussion provides a factual basis for the use of bone marrow transplantation for the treatment of neural injury and neurodegeneration. The methods used with and the utility of the present invention can be shown by the following non-limiting examples and accompanying figures.

Standard molecular biology techniques known in the art and not specifically described were generally followed as in Sambrook et al., Molecular Cloning: A Laboratory Manual, Cold Spring Harbor Laboratory Press, New York (1989), and in Ausubel et al., Current Protocols in Molecular Biology, John Wiley and Sons, Baltimore, Maryland (1989) and in Perbal, A Practical Guide to Molecular Cloning, John Wiley & Sons, New York (1988), and in Watson et al., Recombinant DNA, Scientific American Books, New York and in Birren et al (eds) Genome Analysis: A Laboratory Manual Series, Vols. 1-4 Cold Spring Harbor Laboratory Press, New York (1998) and methodology as set forth in United States patents 4,666,828; 4,683,202; 4,801,531; 5,192,659 and 5,272,057 and incorporated herein by reference. Polymerase chain reaction (PCR) was carried out generally as in PCR Protocols: A Guide To Methods And Applications, Academic Press, San Diego, CA (1990). In-situ (In-cell) PCR in combination with Flow Cytometry can be used for detection of cells containing specific DNA and mRNA sequences (Testoni et al, 1996, Blood 87:3822.)

Standard methods in immunology known in the art and not specifically described are generally followed as in Stites et al.(eds), Basic and Clinical Immunology (8th Edition), Appleton & Lange, Norwalk, CT (1994) and Mishell and Shiigi (eds), Selected Methods in Cellular Immunology, W.H. Freeman and Co., New York (1980).

### EXAMPLES

### TREATMENT OF STROKE (RAT) WITH INTRACERBRAL TRANSPLANTATION OF MSC

**Description of intracerebral transplantation of bone marrow derived MSCs after cerebral ischemia in the rat:** Adult male Wistar rats were used in this study (n=28). Rats were subjected to middle cerebral artery occlusion for two hours using the intraluminal occlusion model. Experimental groups include: (Control) MCAo alone without MSC transplantation (n=8). Injection into the ischemic boundary zone (IBZ) at 24 hours after MCAo of Group 2. Phosphate buffered saline (n=4): Group 3. Non NGF cultured bone marrow MSCs (n=8); Group 4. NGF cultured MSCs (n=8). Approximately 4x10⁴ cells in 10µl total fluid volume were transplanted. Rats received grafts and were sacrificed 14 days after MCAo.

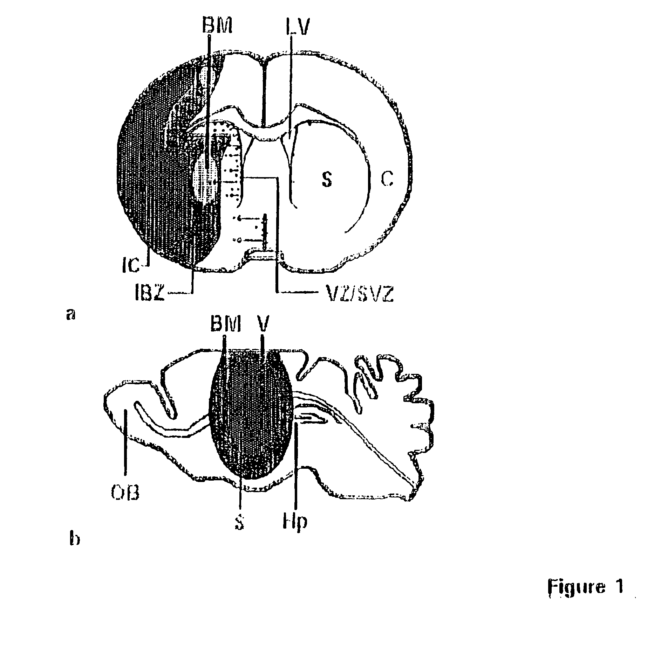

**Behavioral Outcome Measurements:** Behavioral data from the battery of functional tests (rotarod, adhesive-removal and neurological severity score tests) demonstrated that motor and somatosensory functions were impaired by the ischemic insult. No significant differences of the rotarod, adhesive-removal and NSS tests were detected among groups prior to surgery and before transplantation. Significant recovery of somatosensory behavior (p<0.05) and NSS (p<0.05) were detected in animals transplanted with MSCs compared with MCAo alone animals (Figures 1a, c). Animals that received MSCs cultured with NGF displayed significant recovery in motor (p<0.05), somatosensory (p<0.05) and NSS (p<0.05) behavioral tests at 2 weeks post-transplantation with NGF, compared with transplantation of MSCs alone. Figures 1a, b, c show data from the adhesive -removal test, the rotorod-motor test and the neurological severity score (NSS), respectively. These data clearly demonstrate that treatment of stroke with intracranial transplantation of MSCs provides significant therapeutic benefit and that MSCs when cultured in NGF provides superior therapeutic benefit to MSCs cultured without NGF, as indicated in the motor test data (Figure 1b).

### TREATMENT OF STROKE (MOUSE) WITH INTRACEREBRAL TRANSPLANTATION OF MSC

**Intrastriatal transplantation of MSCs into mice after stroke: Embolic MCAo and transplantation.** Experimental adult mice (C57BL/6J, weighing 27-35 g) were subjected to MCAo and transplanted with MSCs (n=5). Control mice were subjected to MCAo alone (n=8); injection of PBS into the ischemic striatum (n=5); and transplantation of MSCs into the normal striatum (n=5). MCAo was induced using an embolic model developed in our laboratory (Zhang et al., 1997). Briefly, using a facemask, mice were anesthetized with 3.5% halothane and anesthesia was maintained with 1.0% halothane in 70% N₂O and 30% O₂. A single intact fibrin-rich in 24 hour old homologous clot (8 mm x 0.000625 mm², 0.18 :I) was placed at the origin of the MCA via a modified PE-50 catheter. Surgical and physiological monitoring procedures were identical to those previously published (Zhang et al., 1997). Four days after MCAo (n=18), mice were mounted on a stereotaxic frame (Stoelting Co. Wood Dale, IL). Using aseptic technique, a burr hole (1 mm) was made on the right side of the skull to expose the dura overlying the right cortex. Semisuspended MSCs (1x10⁵ in 3 :I PBS) were slowly injected over a 10-minute period into the right striatum (AP=0 mm, ML=2.0 mm, and DV=3.5 mm from the bregma). This position approximates the ischemic boundary zone in the striatum. The needle was retained in the striatum for an additional 5 minutes interval to avoid donor reflux. Mice were sacrificed at 28 days after stroke.

**Behavioral Testing:** Each mouse was subjected to a series of behavioral tests (Rotarod-motor test, Neurological severity score) to evaluate various aspects of neurological function by an investigator who was blinded to the experimental groups. Measurements were performed prior to stroke and at 28 days after stroke.

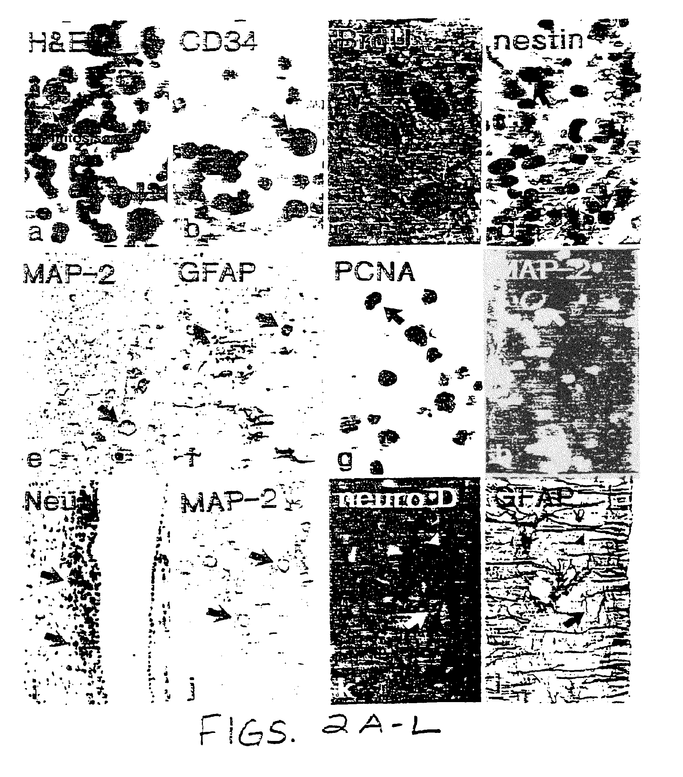

**Results:** BrdU reactive MSCs survived and migrated a distance of approximately 2.2 mm from the grafting areas toward the ischemic areas. BrdU reactive cells expressed of neuronal (∼ 1 % NeuN) and astrocytic proteins (∼8 % GFAP). Functional recovery from a rotarod test (p<0.05) and modified neurological severity score tests (NSS, including motor, sensory and reflex, p<0.05) were significantly improved in the mice receiving MSCs compared with MCAo alone (Figure 2). Figure 2 shows that mice treated with transplanted MSC exhibit a significant improvement in the duration on the rotarod (Figure 2) and how an improved neurological function (Figure 2) compared to vehicle treated animals. The findings suggest that the intrastriatal transplanted MSCs survive in the ischemic brain and improve functional recovery of adult mice.

### TREATMENT OF STROKE (MOUSE) WITH INTRAVASCULAR ADMINISTRATION OF MSC

### Description of experiments:

Experiments were performed on adult male Wistar rats (n=30) weighing 270 to 290 g. In all surgical procedures, anesthesia was induced in rats with 3.5% halothane, and maintained with 1.0% halothane in 70% N₂O and 30% O₂ using a face mask. The rectal temperature was controlled at 37°C with a feedback regulated water heating system. Transient MCAo was induced using a method of intraluminal vascular occlusion, as described above. Two hours after MCAo, reperfusion was performed by withdrawal of the suture until the tip cleared the internal carotid artery.
**(a-intracarotid administration of MSCs)** Intra-carotid transplantation of MSCs was carried out at 24 hours after MCAo (n=23) A modified PE-50 catheter was advanced from the same site of this external carotid artery into the lumen of the internal carotid artery until it rested 2 mm proximal to the origin of the MCA (Figure 1). Approximately 2x10⁶ MSCs in 200 µl PBS (n=6) or Control fluid (200 µl PBS, n=8) were injected over a 10-minute period into each experimental rat. Immunosuppressants were not used in any animal. All rats were sacrificed at 14 days after MCAo.
**(b-Intravenous administration of MSCs)** For intravenous administration of MSCs, a femoral vein was cannulated and either 1,5x10^6 MSCs or 3x10^6 MSCs were injected.

**Behavioral tests and immunohistochemistry:** Each rat was subjected to a series of behavioral tests (NSS and adhesive removal test) to evaluate neurological function before MCAo, and at 1, 4, 7 and 14 days after MCAo. Single and double immunohistochemistry were employed to identify cell specific proteins of BrdU reactive MSCs.

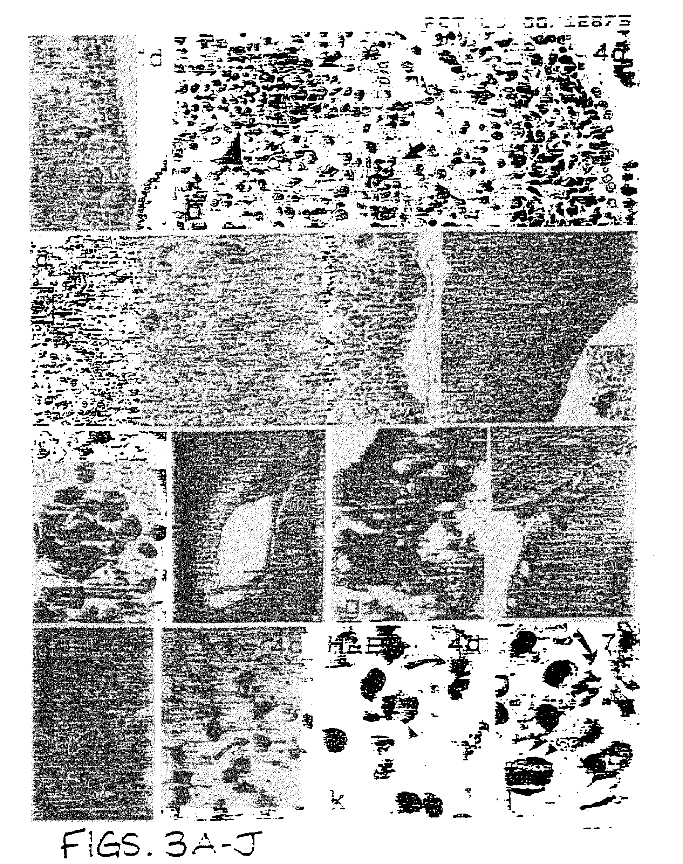

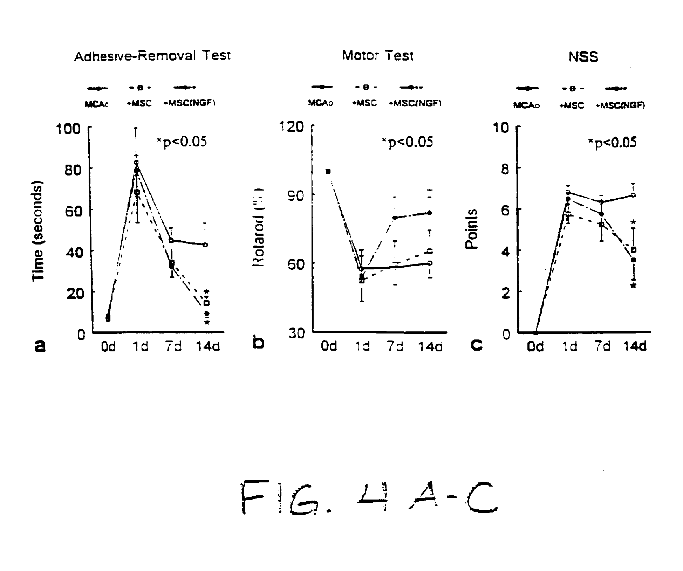

**Results :** For intrarterial administration, BrdU reactive cells (∼21% of 2 x 10⁶ transplanted MSCs) distributed throughout the territory of the MCA by 14 days after ischemia. Some BrdU reactive cells expressed proteins characteristic of astrocytes (glial fibrillary acidic protein, GFAP) and neurons (microtubule associated protein-2, MAP-2). Rats with MSC intra-arterial transplantation exhibited significant improvement on the adhesive-removal test (p<0.05) (Figure 3) and the modified neurological severity scores (p<0.05) (Figure 3) at 14 days, compared with controls. The data for intravenous administration of MSCs were very similar, in that significant functional improvement was present with rats treated with MSCs compared to placebo treated rats. Figure 4 shows functional data from rats administered MSC intravenously compared to control-ischemia rats. A significant improvement is noted in the speed in which the rats removed the sticky tabs from their paws at seven and 14 days after stroke, compared to control animals (Figure 4). The overall neurological function of rats treated with MSCs administered intraarterially was significantly improved compared to control-ischemia rats at 14 days after stroke. The findings suggest that MSCs injected intra-arterially are localized and directed to the territory of MCA and these cells foster functional improvement after cerebral ischemia. In addition, intravenous administration of MSCs also provides a significant improvement in functional outcome. Thus, we have demonstrated that vascular administration is a feasible and effective route of administration of therapeutically beneficial MSCs.

### TREATMENT OF TRAUMATIC BRAIN INJURY (RAT) WITH INTRACERBRAL TRANSPLANTATION OF MSC

**Description:** Experiments were performed on 66 male Wistar rats weighing 250-350 grams. A controlled cortical impact device was used to induce injury (Dixon E et al A controlled cortical impact model of traumatic brain injury in rat. J. Neuroscience Methods 39: 253-262, 1991) Injury was induced by impacting the left cortex with a pneumatic piston containing a 6mm diameter moving at a rate of 4mm/s and producing 2.5mm compression. BrdU labeled MSCs were harvested from donor animals and implanted into the ipsilateral hemisphere, as in the stroke experiments. MSCs were transplanted into brain 24 hours after injury. Rats receiving MSCs were sacrificed at 4 days (n=4), 1 week (n=15), 2 weeks (n=4) and 4 weeks (n=4) after transplantation. Control animals were divided into 3 groups: 1) rats subjected to injury without transplantation and sacrificed at 8 days (n=4) and 29 days (n=4) after injury; 2) animals injected with PBS one day after injury and sacrificed at 4 days (n=4), 7 days (n=4), 14 days (n=4) and 28 days after PBS injection; 3) Sham control rats with craniotomy but no injury or transplantation were sacrificed 8 days (n=4) and 29 days (n=4) after craniotomy.

**Outcome measures (behavior, histology):** An accelerating rotorod test was employed to measure motor function. Measurements were performed at 2, 5, 15, and 29 days after injury. After sacrifice, brain sections were stained with hematoxylin and eosin and double-labeled immunohistochemistry was performed to identify MSC cell type.

**Results:** Histological examination revealed that after transplantation **MSCs** survive, proliferate and migrate towards the injury site. BrdU labeled **MSCs** expressed markers for astrocytes and neurons. Rats transplanted with **MSCs** exhibited a significant improvement in motor function compared with control animals. Our data indicate that intracerebral transplantation of MSC significantly improves neurological function after traumatic brain injury. In a complementary set of experiments, we also treated rats subjected to traumatic brain injury with MSCs; however, in this experiment MSCs were delivered to brain by means of intraarterial (intracarotid artery) administration. Data were similar to intracranial transplantation. MSCs migrated readily into the injured region of brain and these cells expressed protein markers of brain cells (astrocytes, neurons). Thus, our studies indicate that traumatic brain injury can be treated with MSC administers intracerebrally or via a vascular route.

### TREATMENT OF PARKINSONS (MOUSE) WITH INTRACRANIAL TRANSPLANTATION OF MSCs

### Description of MPTP method and results

Adult male C57BU6 mice, 8-12-week-old, weighing 20-35 g, were employed in this study. In order to obtain severe and long lasting lesions, mice were treated with intraperitoneal injections of MPTP hydrochloride (30 mg/kg, Sigma) in saline once a day for seven consecutive days (210 mg/kg total dose). Mice were transplanted with BrdU labeled MSCs (3 x 10⁵/3µl) directly into the right striatum, stereotaxically.

### Behavioral tests

Mice subjected to each MPTP injection, presented and retained behavioral abnormalities (akinesia, postural instability, tremor and rigidity) for several hours, as reported in literature [Heikkila et.al. I., 1989].

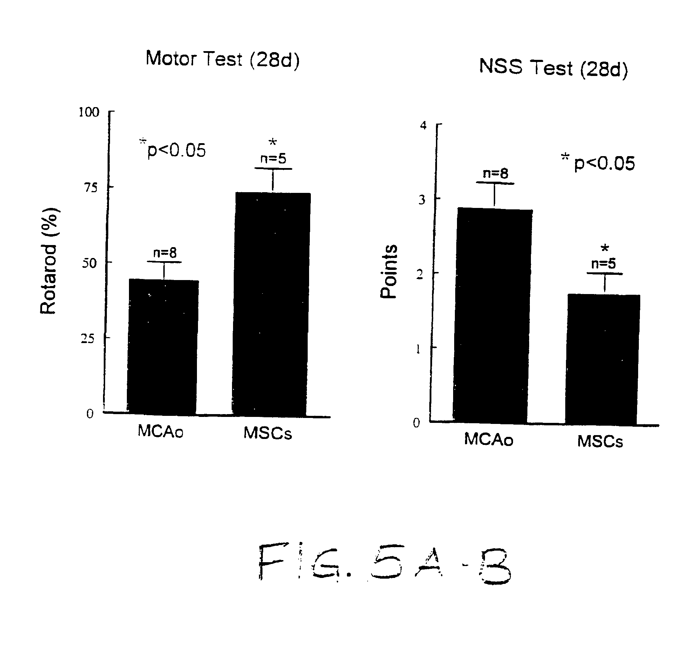

Drug-free evaluation of Parkinsonism using rotarod test was described by Rozas et al. [1997, 1998]. MPTP-PD mice with or without MSC transplantation were tested on a rotarod at an increasing speed (16 rev/minute and 20 rev/minute) after the last MPTP injections (five trials per day to obtain stable values) without any additional enhanced drug injection. A trial was terminated when the mice fell from the rotarod. Significant improvement in motor function (p<0.05) was observed at 35 days after MPTP injection in Parkinson's Disease mice treated with MSC transplantation compared with control MPTP-injected mice alone. Figure 5 hows rotarod data from mice subjected to MPTP neurotoxicity. Two experiments were performed; the mice were placed on the rotorod rotating at 16 rpm or at 20 rpm. The data show that mice treated with MSCs showed a significant increase in duration on the rotarod at both angular velocities compared to MPTP mice given PBS intracerebrally. Mice treated with MSCs cultured with NGF appeared to have incremental benefit compared to MSC treatment, although the differences were not significant.

### Morphological changes:

Viable BrdU immunoreactive cells were identified in the injected area and migrated to variable distances into the host striatum (Figure 1b) at 35 days. Double staining shows that scattered BrdU reactive cells (Figure 1c) express tyrosine hydroxyls (a dopamine marker) immunoreactivity (Figure 1d) within the grafts.

**Conclusions:** These data demonstrate that intracerebral transplantation of MSCs reduces Parkinson disease symptoms in the mouse.

### TREATMENT OF SPINAL CORD INJURY (RAT) WITH INTRALESIONAL TRANSPLANTATION OF MSCs

### Description of spinal cord injury

**Spinal cord injury.** Impact injury was induced using the weight-drop (10g from a height of 25 mm, 'NYU impact' model) to produce a spinal cord injury of moderate severity. Adult male Wistar rats (300 ± 5 g) were anesthetized with pentobarbital (50 mg/kg, intraperitoneally), and a laminectomy was performed at the T9 level.

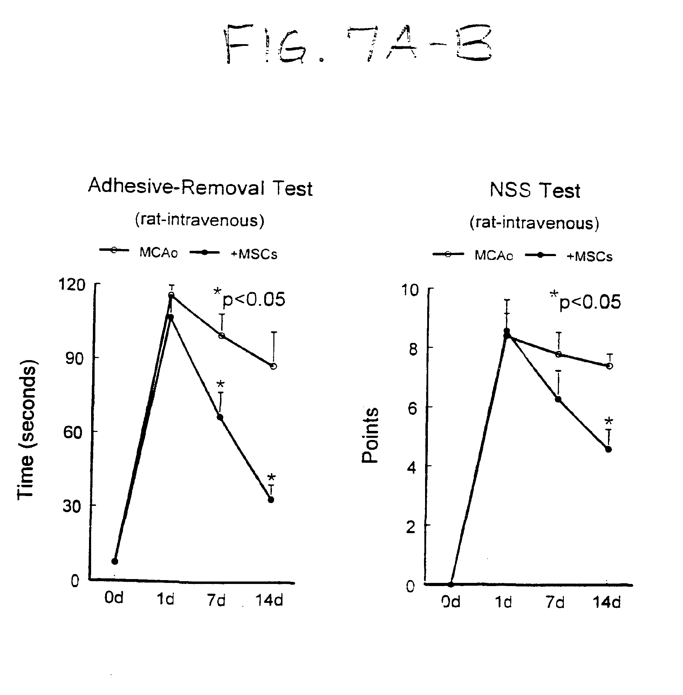

**Transplantation and behavioral testing.** MSCs 2.5 X 10⁵ /4 :I were injected into the epicenter of injury at 7 days after SPI. The Basso-Beattie-Bresnahan (BBB) Locomotor Rating scores were obtained before and after transplantation Basso et al., 1995]. Figure 7 shows data from the BBB test from animals subjected to spinal cord injury and treated with MSC transplantation or simply given the same volume of vehicle. All rats had a score of 21 (normal score) before spinal cord injury and a score of 0 at 6 hours after contusion. In the rats subjected to contusion with PBS injection, scores improved from 6.7 (1 week) to 11.5 (5 weeks). The control group had an early improvement in neurologic function, which plateaus by the third week. The rats subjected to contusion with MSC transplantation had a significantly improved score of 7.0 (1 week) and 15.3 (5 weeks). The MSC treated group exhibited a steady recovery that had not plateaued by the fifth weeks, which was the end point of the experiment. The MSC treated rats had significant improvement on BBB scores with the p-value, 0.01 for overall and each individual time point for treatment effect. In functional terms, the contused rats in the MSC treated group could walk with consistent weight supported plantar steps with forelimb and hindlimb coordination. In contrast, the contused rats in the PBS control group exhibited obvious motor function deficits.

### Histological Analysis

Cells derived from MSCs, identified by BrdU immunoreactivity, survived and were distributed throughout the damaged tissue (T9, Figure 1a) from 1 week to 4 weeks after MSC transplantation. BrdU reactive cells migrated 5 mm both caudal and rostral from the epicenter of transplanted cells (Figure 1b). Figure 2a shows that the antibody against Rip did not react with damaged oligodendrocytes in contused rats with non treated PBS injection. In contrast, after spinal cord injury and MSC transplantation (Figure 2b), intense Rip immunoreactivity clearly demarcated myelinated small and large diameter fibers. Double immunostaining (Figures 2c-d) demonstrates that scattered BrdU reactive cells express the neuronal marker, NeuN.

**Conclusions:** Treatment of moderate to severe spinal cord injury with MSCs transplanted into the site of injury provides significant improvement of motor function. The MSCs express protein markers of neurons and oligodendrocytes, indicating that these cells when placed within the spinal cord acquire characteristics of parenchymal cells.

### NEUROSPHERE (NMC-SPHERE)- A NEW COMPOSITE FOR THE TREATMENT OF CNS INJURY AND DISEASE

### Description of neurosphere experiment

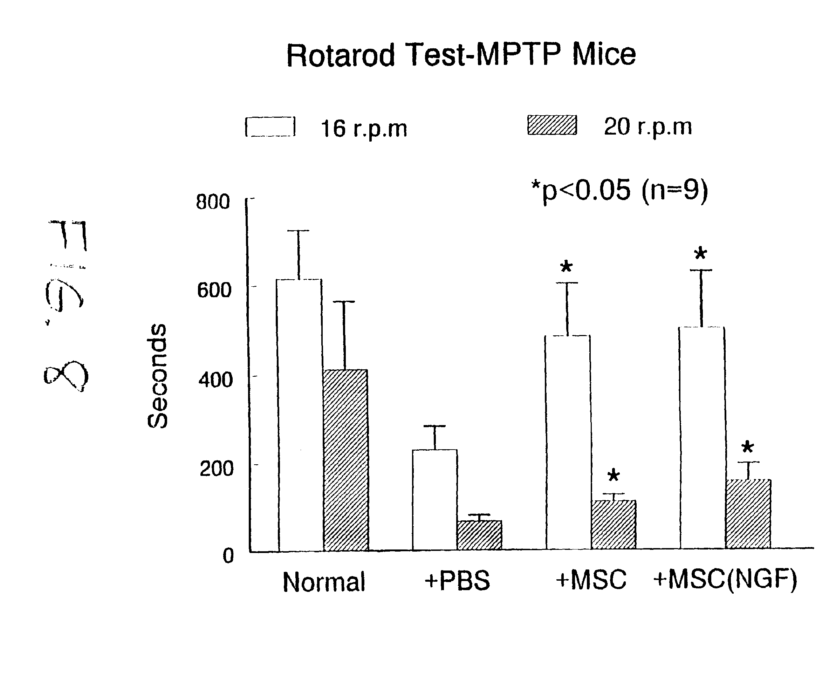

We have employed aggregates, composed of **neural stem cells** from fetal neurosphere, **mesenchymal stem cells** from adult bone marrow and **cerebro-spinal fluid** from adult Wistar rats (called NMCspheres). Fetal brain cells were pre-labeled with 1,1'-dioctadecy-6, 6'-di(4-sulfopheyl)-3,3,3', 3'-tetramethylindocarbocyanine (DiI) and bone marrow mesenchymal cells from adult rats were pre-labeled with 3,3'-dioctadecyloxacarbocyanine perchlorate (DiO) and/or bromodeoxyuridine (BrdU). Using laser scanning confocal microscopy (three-dimensional) and immunohistochemical analysis on paraffin and frozen sections, we identified that:
1. Cell-cell interaction: Within the NMCsphere, cells derived from bone marrow mesenchymal stem cells, rapidly form a scaffold (1 day) and a network (9 days, Figure 8) overtime, in vitro. Figure 8 shows the composite MSC neurosphere nine days after cell-neurosphere integration. The MSC, identified by DiO and BrdU form an axonal-dendritic like network (yellow-green).
2. **Cell-cell interaction:** Within the NMCsphere, cells derived from neural stem cells have a longer life span than within neurosphere alone. The NMCspheres express proteins, e.g., nestin that is normally found in immature neural cells; glial fibrillary acidic protein (GFAP) that is a specific marker for differentiated astrocytes; myelin basic protein (MBP) that is a marker of oligodendrocytes; and neuron-specific class III β-tubulin (TuJ1) that is a marker for immature neurons and microtubule associated protein 2 (MAP-2) that is a marker for neuronal cell bodies and dendrites.
3. **NMCsphere-microenvironment:** The size and structure of the NMCspheres are influenced by the microenvironment of the medium, i.e., they grow better in the IMDM with stem cell factor than with standard DMEM.
4. **Secretion of NMCspheres:** Adding the supernatant from the cultured NMCsphere into the medium DMEM and IMDM for neurospheres and MSCs, respectively, stimulated the growth of both neurospheres and MSCs. Obvious cell-cell connection and proliferation was induced with this supernatant. This suggests the NMCspheres secrete supporting substances for stem cells. These substances can be used to enhance neurogenesis.
5. **Cerebro-spinal fluid** (CSF) provides an optimal microenvironment to form NMCspheres that is superior to conventional medium.

### TREATMENT OF STROKE AND BRAIN TRAUMA WITH NMCsphere

Protocol for MSC & neurosphere transplantation in rats after MCAo and TBI.

### MCAo

BrdU prelabeled MSCs and neurospheres were mixed and cultured in flasks for 7 days. At 24 hours after MCAo, rats were anesthetized with halothane and the composite NMSsphere was injected into brain (n=4). The animals were mounted on a stereotaxic apparatus (Model 51603, Stoelting Co., Wood Dale, IL). Twenty spheres (diameter less than 0.2 mm) in 5 ml PBS were injected vertically by a Hamilton syringe into the right striatum at the coordinates LM=2.5 mm, VD=4.5 mm and AP=0 to the bregma, and into the right cortex at LM=2.5 mm, VD=2 mm and AP=0 mm. This position approximates the ischemic boundary zone. Three microliters of spheres were initially injected into the striatum and 2 ml into the cortex over a 10-minute period in each spot. The needle was retained in the cortex for an additional 5-minute interval to avoid bone marrow reflux from the injected areas to the brain surface. After injection, bone wax (W810, Ethicon) was placed on the skull to prevent the leakage of the solution. Rats were sacrificed at 14 days after MCAo.

### Traumatic Brain Injury (TBI)

BrdU prelabeled MSCs and neurospheres were mixed and cultured in flasks for 7 days. At 4 days after TBI rats (n=4) were anesthetized with chloride hydrate and placed onto the stereotactic frame, and then exposed the previous injured area. A pipette with a glass tip (0.5 mm of diameter) containing 15 prepared mixed NMCspheres (diameter of 0.25 mm) in 20 UL PBS was fixed onto the stereotactic frame. The tip of the needle was inserted at the central site of the injured area, 2.5 mm away from brain surface. Spheres were injected into the brain over 5 minutes, and then kept for an additional 5 minute interval to avoid reflux. In both sets of experiments (stroke and TBI) functional outcome measurements were measured using the rotorod and adhesive removal tests.

**Results:** Functional benefit in both stroke and TBI was evident in rats treated with NMCspheres. These data indicate that NMCspheres can be employed for the treatment of stroke and brain injury. This composite, is a new material with potential for the treatment of CNS injury and neurodegeneration.

### DESCRIPTION OF NOVEL MEDIUM (WITH AND WITHOUT GROWTH FACTORS) EMPLOYED FOR THE CULTURING OF MSCs FOR THE TREATMENT OF NEURAL INJURY AND NEURODEGERATION

Primary bone marrow cells were obtained at 48 hours after treating adult Wistar rats with 5-fluorouracil (5-FU, 150 mg/kg) and cultured in the Iscove=s Modified Dulbecco=s Medium (IMDM) supplemented with 10% fetal bovine serum (FBS) and stem cell factor (100 ng/ml). Adherent MSCs were resuspended in fresh IMDM with nerve growth factor (NGF, 200 ng/ml), brain-derived neurotrophic factor (BDNF, 100 ng/ml) and epidermal growth factor (EGF, 20 ng/ml) up to one month. Control MSCs were cultured in the IMDM without neural growth factors. Antibodies against neuronal nuclei (NeuN), microtubule associated protein-2 (MAP-2) and glial fibrillary acidic protein (GFAP) were used for immunocytochemical identification of cultured cells.

The data indicates that cells derived from adult bone marrow stem and progenitor cells can grow in large quantities in culture and express proteins characteristic of neurons and astrocytes. Neurotrophic growth factors enhance the neural expression of cells derived from bone marrow cells in vitro. Immunocytochemical staining shows that control MSCs without neurotrophic growth factors express the neuronal NeuN (∼1%, Figure 1 a) and astrocytic GFAP (∼3%, Figure 1b). However, MSCs treated with neurotrophic growth factors (e.g., NGF) express neuronal NeuN (∼3%, Figure 1c) and astrocytic GFAP (∼30%, Figure 1d).

Bromodeoxyuridine (BrdU, 3 Fg/ml), which is incorporated into dividing cells, and identifies newly formed DNA, was added to the medium at 72 hours before transplantation. Using immunoperoxidase with 3.3'-diaminobenzidine (DAB, brown) and counter staining by hematoxylin, bone marrow cells are identified by the antibody against BrdU. The number of MSCs labeled with BrdU is ∼90% in vitro.

### DISCUSSION

The data demonstrate that cultured adult bone marrow cells particularly marrow stromal cells (MSCs) survive and differentiate into parenchymal like cells in the adult rodent brains after ischemia, brain and spinal cord trauma, and Parkinson's disease, and that bone marrow promotes prominent proliferation, differentiation and migration of VZ/SVA NSCs.

Pluripotent bone marrow cells become glia in normal rat brain (Azizi et al., 1998), and facilitate cell proliferation and cell-specific differentiation after MCAo. The bone marrow transplantation experiment requires a sensitive means of monitoring the fate of the bone marrow cells. Help came from the bone marrow cells carrying tracers and markers, such as BrdU, CD34, nestin, PCNA. Pluripotent hematopoietic stem cells and mesenchymal stem cells from the adult bone marrow exposed to the new ischemic microenvironment after MCAo are triggered to proliferate and differentiate into neuronal (MAP-2, NeuN) and glial cell (GFAP) phenotypes. Fresh bone marrow or stroma humoral factors are also be a source of differentiating factors and provides the chemotatic microenvironment to enhance the proliferation, migration and differentiation of neural stem cells from VZ/SVZ.

The VZ/SVZ of the mammalian forebrain is a region of germinal matrices that develops late in gestation, enlarges, and then diminishes in size, but persists in a vestigial form throughout life (Gage 1998). In the normal adult brain, the absence of forebrain neuronal production reflects not a lack of appropriate neural stem cells, but rather a tonic inhibition and/or a lack of postmitotic trophic and migratory support. Although the signals that trigger the quiescent CNS stem cells within the normal VZ/SVZ to enter the cell cycle have yet to be resolved, the data show that a lesioned CNS is a different environment than an intact CNS and markedly alters the terminal differentiated phenotype of the neural stem cells. Importantly, the VZ/SVZ in the adult forebrain is not a passive ischemia-threatened zone, located far from the ischemic areas (FIGURES 3F-H), but is an active tissue providing cells to reconstruct brain. VZ/SVZ cells proliferate and differentiate into neuronal and glial phenotypes after MCAo. The survival of neurons arising from adult NSCs is dictated by both the availability of a permissive pathway for migration and the environment into which migration occurs. New neurons depart the VZ/SVZ to enter the brain parenchyma via radial guide fibers, which emanate from cell bodies in the ventricular ependyma in adult rat (FIGURES 2K-L), and provide a permissive pathway for migration as found during development (Rakic 1972). Mitosis within the graft and VZ/SVZ show that ischemic injured brain together with the transplanted cells reverts to an early stage of development to promote repair. The data are consistent with the observation that adult brain can form new neurons (Gage 1998).

In summary, the data indicate that intracerebral and intravascular bone marrow transplantation after stroke neural injury and Parkinson's disease significantly improves functional recovery. Transplantation also enhances the proliferation and differentiation of exogenous bone marrow stem cells and endogenous NSCs. Bone marrow aspirations and biopsies have been employed in the diagnosis and treatment of clinical diseases. Bone marrow transplantation provides a new avenue to induce plasticity of the injured brain and spinal cord and provides a therapeutic strategy for treatment of neural injury and neurodegeneration.

In addition, a new substance is identified, a composite of **MSCs** and neurospheres, which when transplanted into brain after stroke or trauma, Improves functional recovery.

Throughout this application, various publications are referenced by author and year. Full citations for the publications are listed below.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, the invention may be practiced otherwise than as specifically described.

### REFERENCES

Burke and Olson, "Preparation of Clone Libraries in Yeast Artificial-Chromosome Vectors" in Methods in Enzymology, Vol. 194, "Guide to Yeast Genetics and Molecular Biology", eds. C. Guthrie and G. Fink, Academic Press, Inc., Chap. 17, pp. 251-270 (1991).
Capecchi, "Altering the genome by homologous recombination" Science 244:1288-1292 (1989).
Davies et al., "Targeted alterations in yeast artificial chromosomes for inter-species gene transfer", Nucleic Acids Research, Vol. 20, No. 11, pp. 2693-2698 (1992).
Dickinson et al., "High frequency gene targeting using insertional vectors", Human Molecular Genetics, Vol. 2, No. 8, pp. 1299-1302 (1993).
Duff and Lincoln, "Insertion of a pathogenic mutation into a yeast artificial chromosome containing the human APP gene and expression in ES cells", Research Advances in Alzheimer's Disease and Related Disorders, 1995.
Huxley et al., "The human HPRT gene on a yeast artificial chromosome is functional when transferred to mouse cells by cell fusion", Genomics, 9:742-750 (1991).
Jakobovits et al., "Germ-line transmission and expression of a human-derived yeast artificial chromosome", Nature, Vol. 362, pp. 255-261 (1993).
Lamb et al., "Introduction and expression of the 400 kilobase precursor amyloid protein gene in transgenic mice", Nature Genetics, Vol. 5, pp. 22-29 (1993).
Pearson and Choi, Expression of the human b-amyloid precursor protein gene from a heast artificial chromosome in transgenic mice. Proc. Natl. Scad. Sci. USA, 1993. 90:10578-82.
Rothstein, "Targeting, disruption, replacement, and allele rescue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301 (1991).
Schedl et al., "A yeast artificial chromosome covering the tyrosinase gene confers copy number-dependent expression in transgenic mice", Nature, Vol. 362, pp. 258-261 (1993).
Strauss et al., "Germ line transmission of a yeast artificial chromosome spanning the murine a, (I) collagen locus", Science, Vol. 259, pp. 1904-1907 (1993).
Gilboa, E, Eglitis, MA, Kantoff, PW, Anderson, WF: Transfer and expression of cloned genes using retroviral vectors. BioTechniques 4(6):504-512, 1986.
Cregg JM, Vedvick TS, Raschke WC: Recent Advances in the Expression of Foreign Genes in Pichia pastoris, Bio/Technology 11:905-910, 1993.
Culver, 1998. Site-Directed recombination for repair of mutations in the human ADA gene. (Abstract) Antisense DNA & RNA based therapeutics, February, 1998, Coronado, CA.
Huston et al, 1991 "Protein engineering of single-chain Fv analogs and fusion proteins" in Methods in Enzymology (JJ Langone, ed.; Academic Press, New York, NY) 203:46-88.
Johnson and Bird, 1991 "Construction of single-chain Fvb derivatives of monoclonal antibodies and their production in Escherichia coli in Methods in Enzymology (JJ Langone, ed.; Academic Press, New York, NY) 203:88-99.
Mernaugh and Memaugh, 1995 "An overview of phage-displayed recombinant antibodies" in Molecular Methods in Plant Pathology (RP Singh and US Singh, eds.; CRC Press Inc., Boca Raton, FL) pp. 359-365.
Snyder EY, Park KI, Flax J, Liu S, Rosario C, Yandava BD, Aurora Y. Potential of neural "stem-like" cells for gene therapy and repair of the degenerating central nervous system, Adv Neurol;72:121-132, (1997).
Goto et al., Exp. Neurol. 147:503-509, (1997).
Geiger H, Sick S, Bonifer C, Müller AM. Globin gene expression is reprogrammed in chimeras generated by injecting adult hematopoietic stem cells into mouse blastocysts. Cell.; 93:1055-1065 (1998).
Gage FH, "Stem cells of the central nervous system" Curr. Opin. Neurobiol. 8:671-676, (1998).
Kempermann G, Gage FH, "Closer to neurogenesis in adult humans", Nat. Med. 4:555-557, (1998).

## Claims

1. Use of bone marrow stromal cells in the manufacture of a medicament for the treatment of stroke by transplanting the cells intravascularly by intracarotid administration or intravenous administration.

2. Use of bone marrow stromal cells according to claim 1 to activate endogenous central nervous system stem cells to differentiate into neurons in an injured brain.

## Patentansprüche

1. Verwendung von Stromazellen aus dem Knochenmark zur Herstellung eines Medikaments zur Behandlung von Schlaganfall durch intravaskuläres Transplantieren der Zellen mittels intrakarotider Verabreichung oder intravenöser Verabreichung.

2. Verwendung von Stromazellen aus dem Knochenmark nach Anspruch 1 zur Aktivierung endogener Stammzellen des zentralen Nervensystems zur Differenzierung in Neuronen in einem verletzten Hirn.

## Revendications

1. Utilisation de cellules stromales de la moelle osseuse dans la fabrication d'un médicament pour le traitement de l'accident vasculaire cérébral par transplantation des cellules par voie intravasculaire par administration intracarotidienne ou par administration intraveineuse.

2. Utilisation de cellules stromales de la moelle osseuse selon la revendication 1, pour activer des cellules souches endogènes du système nerveux central à se différencier en neurones dans un cerveau atteint d'une lésion.
